# EUROPEAN PATENT SPECIFICATION

(11) **EP 2 677 314 B1**
(45) Date of publication and mention of the grant of the patent: **12.07.2017**
(21) Application number: 13002701.4
(22) Date of filing: 24.05.2013
(51) Int. Cl.: G01N 33/497

(54) **Chemical sensor in a portable electronic device**
Chemischer Sensor in einer tragbaren elektronischen Vorrichtung
Capteur chimique dans un dispositif électronique portable

(30) Priority: 21.06.2012 US 201213529062
(43) Date of publication of application: 25.12.2013
(73) Proprietor: Sensirion AG, 8712 Stäfa (CH)
(72) Inventor: Mayer, Felix, CH-8712 Stäfa (CH); Graf, Markus, CH-8005 Zürich (CH); Bürgi, Lukas, CH-8049 Zürich (CH)
(74) Representative: Toleti, Martin

(56) References cited:
- WO-A1-2008/020223
- US-A- 4 278 636
- US-A1- 2012 055 726
- MUEZZINOGLU M K ET AL: "Acceleration of chemo-sensory information processing using transient features", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 137, no. 2, 2 April 2009 (2009-04-02) , pages 507-512, XP026029048, ISSN: 0925-4005, DOI: 10.1016/J.SNB.2008.10.065 [retrieved on 2009-03-21]
- Michael Karst ET AL: "Humidity & Temperature Sensors in Mobile Phones", Wireless communication alliance event, 18 April 2012 (2012-04-18), pages 1-18, XP055069470, Retrieved from the Internet: URL:http://www.wca.org/event_archives/2012 /Sensirion_WCA_April2012_Mobile_Sensors.pd f [retrieved on 2013-07-03]
- ANAND D MANE ET AL: "Explosive detection with mobile telephony an attempt towards a safe ambience", SIGNAL PROCESSING, COMMUNICATION, COMPUTING AND NETWORKING TECHNOLOGIES (ICSCCN), 2011 INTERNATIONAL CONFERENCE ON, IEEE, 21 July 2011 (2011-07-21), pages 187-191, XP031940738, DOI: 10.1109/ICSCCN.2011.6024541 ISBN: 978-1-61284-654-5

## Description

### Technical Field

The present invention relates to a portable electronic device and to a method for operating a portable electronic device.

### Background Art

Today's smart phones or tablet computers contain a couple of sensors such as, for example, a gyroscope or an acceleration sensor used for detecting an orientation of the device display for having the content to be displayed adapted to such orientation.

In general, chemical sensors are known for detecting an analyte in a gas or a liquid, collectively denoted as fluid. When integrating a chemical sensor into a portable electronic device, it is a challenge to meet a point in time for taking a reading by the chemical sensor at which point in time the chemical sensor already is sufficiently exposed to the fluid to be analyzed.

In WO 2008/020223 A1, a remote sensor platform for asset tracking is introduced that monitors a context of a local environment to conserve power. Primary sensors monitor local environment stimuli such as temperature, pressure or illumination. A low-power processor uses the primary sensors to monitor the environment and thereby determine whether to activate a secondary high power sensor, such as a GPS unit or a humidity or gas sensor. The low power processor may be triggered by the primary sensors and may use configurable rules for decision making. It may log exceptions and sensor data for further decision making. A high-power processor sends sensor data via a reporting means to a server using secondary configurable rules conditionally on the primary and the secondary sensor inputs. The server can update the rules.

According to "Acceleration of chemo-sensory information processing using transient features" Meuzzi-noglu M. K. et al, in Sensors and Actuators B, vol. 137, 2 April 2009, pages 507-512, a snapshot from a steady-state response of chemical sensors conveys, on average, more mature and relevant information regarding the analyte than a snapshot from a transient can provide. Nevertheless, time constraints in many applications make it infeasible to wait for and extract steady-state features. Substituting them by transient ones is a viable solution to accelerate odor processing. Based on measurements recorded from metal-oxide sensors, a correlation between a transient feature and the steady-state resistance is observed in response to a fixed analyte concentration. This correlation is used to expedite standard quantification and classification substantially while ensuring a performance that the steady-state feature can provide.

### Disclosure of the Invention

Hence, it is desired to determine a point in time suitable for taking a chemical sensor reading in a portable electronic device.

According to a first aspect of the present invention, a portable electronic device is provided comprising a chemical sensor being sensitive to at least one analyte, and a trigger sensor providing a signal. A control unit is provided for triggering a sampling of a chemical sensor reading subject to the signal of the trigger sensor.

A semiconductor chip contains the chemical sensor, wherein the trigger sensor includes a humidity sensor which is contained in the semiconductor chip.
- [not claimed] a semiconductor chip contains the chemical sensor and an integrated circuit arrangement, wherein the trigger sensor includes a temperature sensor which temperature sensor is arranged in the semiconductor chip for determining a temperature of at least a part of the integrated circuit arrangement;
- [not claimed] the trigger sensor includes a flow sensor for determining a flow of a gas across the sensitive layer, and in particular the chemical sensor comprises a sensitive layer being sensitive to the at least one analyte, and a heater for heating the sensitive layer, and the flow sensor includes one of a temperature sensor for determining a temperature of the sensitive layer of the chemical sensor and a determination unit for determining an electrical operating value of the heater of the chemical sensor;

Preferred embodiments of the first aspect contain one or more of the following features:
- a housing and a compartment in the housing, which compartment comprises an access opening to an environment of the portable electronic device, and wherein the chemical sensor is arranged in the compartment;
- the trigger sensor is arranged in the compartment;
- the chemical sensor comprises a sensitive layer and a heater for heating the sensitive layer, wherein the sensitive layer comprises a metal-oxide material, and wherein the metal-oxide material includes one or more of tin oxide, zinc oxide, titanium oxide, tungsten oxide, indium oxide and gallium oxide;
- the control unit is adapted for triggering a sampling of the chemical sensor reading subject to the signal of the trigger sensor exceeding a threshold;
- a third sensor supplying a signal, wherein the third sensor is one of a humidity sensor, a temperature sensor, a flow sensor, a gyroscope, an accelerometer, a microphone and a pressure sensor, and wherein the control unit is adapted to trigger the sampling of the chemical sensor reading subject to the signal of the trigger sensor and subject to the signal of the third sensor;
- the chemical sensor is adapted and arranged for detecting one or more of a presence and a concentration of the at least one analyte in an exhalation airflow of a user of the portable electronic device;
- the portable electronic device is one of a mobile phone, a handheld computer, an electronic reader, a tablet computer, a game controller, a pointing device, a photo or a video camera, a computer peripheral.

According to another aspect of the present invention [not claimed] a portable electronic device is provided, comprising a chemical sensor being sensitive to at least one analyte and providing an output signal, and a control unit for triggering a sampling of a chemical sensor reading subject to the output signal of the chemical sensor.

In a preferred embodiment [not claimed], the chemical sensor comprises multiple cells being sensitive to different analytes, and wherein the control unit is adapted to trigger the sampling of the chemical sensor reading subject to the output signal of at least one of the chemical sensor cells.

According to a further aspect of the present invention, a method is provided for operating a portable electronic device containing a chemical sensor being sensitive to at least one analyte and containing a trigger sensor, the method comprising monitoring a signal supplied by the trigger sensor, and subject to the signal of the trigger sensor taking a chemical sensor reading for detecting one or more of a presence and a concentration of the at least one analyte in a fluid supplied to the chemical sensor.

Preferred embodiments of this aspect contain one or more of the following features:
- the signal of the trigger sensor is monitored in response to a user request for taking the chemical sensor reading;
- a heater of the chemical sensor is activated in response to a user request for taking the chemical sensor reading;
- a heater of the chemical sensor is activated subject to the signal of the trigger sensor.

According to another aspect of the present invention [not claimed], a method is provided for operating a portable electronic device containing a chemical sensor being sensitive to at least one analyte and providing an output signal, the method comprising monitoring the output signal of the chemical sensor, and subject to the output signal of the chemical sensor taking a chemical sensor reading for detecting one or more of a presence and a concentration of the at least one analyte in a fluid supplied to the chemical sensor.

According to a further aspect of the present invention, a computer program product is provided comprising a computer readable medium containing computer program code for implementing a method according to any one of the previous embodiments when being executed on a control unit.

Other advantageous embodiments are listed in the dependent claims as well as in the description below.

### Brief Description of the Drawings

Embodiments of the present invention will be described in more detail with reference to the annexed drawings, wherein the Figures show:
FIG. 1 a usage scenario with a mobile phone according to an embodiment of the present invention,
FIG. 2 a block diagram of a portable electronic device according to an embodiment of the present invention,
FIG. 3 a cross section of a part of a housing of a portable electronic device according to an embodiment of the present invention,
FIG. 4 a top view on a semiconductor chip contributing to a portable electronic device according to an embodiment of the present invention,
FIG. 5 a cross sectional cut of a chemical sensor integrated in a semiconductor chip as used in a portable device according to an embodiment of the present invention,
FIG. 6 a block diagram of a portable electronic device according to an embodiment of the present invention, and
FIG. 7 a flow diagram representing a method according to an embodiment of the present invention.

### Modes for Carrying Out the Invention

A chemical sensor preferably comprises a layer that is sensitive to one or more analytes a presence and/or a concentration of which is desired to be detected in a gas supplied to the chemical sensor. There may be a single sensitive layer made from a uniform material for interacting with the one or more analytes. Or there may be multiple sensitive layers made from different materials, for example, for interacting with different analytes. The chemical sensor performs a detection of chemical substances or compounds which are also denoted as analytes contained in a gas, or possibly in a fluid including liquids. Analytes may be, for example, CO2, NOX, ethanol, CO, ozone, ammonia, formaldehyde, or xylene without limitation.

In a preferred embodiment, the sensitive layer may contain a metal-oxide material, and in particular a semiconducting metal oxide material. Such metal oxide material may include one or more of tin oxide, zinc oxide, titanium oxide, tungsten oxide, indium oxide and gallium oxide. Such metal oxides may be used for the detection of analytes such as VOCs, carbon monoxide, nitrogen dioxide, methane, ammonia or hydrogen sulphide. Metal-oxide sensors are based on the concept that gaseous analytes interact with the metal oxide layer at elevated temperatures of the sensitive layer in the range of more than 100° Celsius, and specifically between 250°C and 350°Celsius. As a result of the catalytic reaction, a conductivity of the sensitive layer may change which change can be measured. Hence, such chemical sensors are also denoted as high temperature chemoresistors for the reason that a chemical property of the analyte is converted into an electrical resistance at high temperatures of the sensitive layer.

Hence, a chemical sensor may in one embodiment comprise at least one sensor material, e.g. in form of a layer, an analyte may interact with and as such modify an electrical property of the sensor material such as its electrical conductance. Then, the electrical property of a combination of the analyte and the sensor material is measured and allows a conclusion as to a presence of the analyte, such as by way of comparison to a property of the sensor material measured without the presence of the analyte. It is noted that for different analytes - which may be different chemical elements or chemical compounds - it is not required to always measure the same property per analyte. Different properties may be measured for different analytes.

Specifically, the chemical sensor may be a gas sensor for detecting one or more substances in a gas, and specifically in the air surrounding the portable electronic device. Hence, in a sample application it may be of interest to identify if such air may contain analytes the chemical sensor is prone to. Other applications may include the detection of toxic gases, the detection of ethanol in a users breath, or the detection of other substances.

In case the chemical sensor is sensitive to multiple different analytes the chemical sensor may be embodied as a sensor array. Such sensor array may comprise multiple sensor cells, wherein each sensor cell may provide a sensor material, e.g. in form of a layer which is also denoted as sensitive layer, an analyte may interact with. In response to the interaction an electrical property of the sensor material such as its electrical conductance, may change which principle preferably is applied in metal oxide chemical sensors. In such scenario, the sensor array may comprise a single heater for all sensor cells, or may comprise multiple heaters, wherein each heater may be assigned for heating a group of sensor cells or an individual sensor cell, or the corresponding sensitive layer respectively.

In another embodiment, the chemical sensor may comprise a single sensor cell, e.g. with a single layer, which however, may be sensitive to multiple different analytes under different operating conditions. For example, the sensor cell may mainly be sensitive to a first analyte x when being heated to a first temperature tₓ, and may mainly be sensitive to a second analyte y when being heated to a second temperature ty which is different from the first temperature tₓ. In another variant, a sensor array may comprise multiple sensor cells wherein at least one of the multiple sensor cells - and in another variant preferably all of the multiple sensor cells - is/are designed such that such cell/s may mainly be sensitive to different analytes under different operating conditions such as under different temperatures. In such specific embodiment, each of such sensor cell/s may be provided with an individual heater.

The chemical sensor preferably is arranged inside a housing of the portable electronic device. An access opening may be provided in the housing for exposing the chemical sensor to a fluid to be analyzed, and specifically to a gas to be analyzed.

Hence, any portable electronic device such as a mobile phone, and in particular a smart phone, a handheld computer, an electronic reader, a tablet computer, a game controller, a pointing device, a photo or a video camera, or a computer peripheral - which listing is not limited - may in addition to its original function provide chemical information as to its environment. The user may learn about chemical substances and compositions present in the devices surroundings and/or his/her breath, and may use, transmit or else further analyse such information. For the reason that such portable electronic device typically includes interfaces to a remote infrastructure, such information may be transmitted elsewhere and used elsewhere. In an alternative, the user himself/herself may benefit from the information provided by the chemical sensor in that actions can be taken in response to detected analytes, including but not limited to analytes representing toxic substances. Such portable electronic device may primarily be designed for computing and/or telecommunication and/or other tasks in the IT arena, and now be enhanced by a function of providing chemical information.

The chemical sensor preferably may be arranged in a compartment of the housing wherein the access opening may provide access to the chemical sensor in the compartment. Therefore, a gas to be analyzed is required to diffuse into the compartment. Such process may take some time. If, however, a chemical sensor reading is taken too early at e.g. a point in time when the gas of interest may not have yet reached the chemical sensor or may have reached the chemical sensor only to an insufficient degree, the chemical sensor reading may not be correct and reflect a measurement value not representing the current analyte concentration in the gas. Hence, the timing of taking a reading by the chemical sensor is important.

The terms "taking a chemical sensor reading" and "triggering a sampling of a chemical sensor reading" may include accepting or using a measurement value supplied by the chemical sensor, or may include measuring a value and accepting or using said value. Acceptance or use of a reading, i.e. is a measurement value, includes a usage of the chemical sensor reading for the purpose the reading was taken, e.g. for displaying the reading to the user, for further processing of the value, for forwarding the value etc. In case the chemical sensor continuously supplies readings in form of an output signal - e.g. when a voltage is permanently applied to a sensitive layer of a chemical sensor - taking the reading including triggering taking the reading may include utilizing the present reading for the very purpose of the reading or utilizing a reading defined with respect to the time of the trigger while other readings outside such specification may be discarded. However, taking a reading or triggering a sampling of a reading shall not be limited to the usage of a single reading at the trigger point in time. Instead, taking the reading may include the usage of multiple present, past and/or future readings, for example, for building an average value amongst the readings taken according to a specification. In another embodiment, at the trigger point in time an integration of readings of the chemical sensor is started, for example, for a defined period in time, which integration value may finally be the value of interest. In case the chemical sensor is not continuously operative in providing measurement values, taking the reading including triggering taking the reading may include activating a measurement, which, for example, may include applying a voltage to the sensor layer, etc., and making use of the measured value.

The point in time at which a chemical sensor reading is taken depends on a signal of at least a trigger sensor. The trigger sensor may not necessarily be solely provided for the purpose for triggering the taking of a reading of the chemical sensor. Instead the trigger sensor may be represented by a sensor present in the portable electronic device for a different purpose and may be used additionally for triggering the taking of the reading of the chemical sensor. The trigger sensor is provided in the portable electronic device, and is a humidity sensor. The trigger sensor preferably is arranged and designed for allowing a conclusion that based on its signal the fluid to be analyzed is believed to have sufficiently diffused into the compartment yet, such that the chemical sensor is sufficiently exposed to the fluid for detecting the presence and/or concentration of an analyte in the gas. Hence, it is preferred that such trigger sensor may either detect the presence of the gas, preferably in a different way the chemical sensor does, or may allow drawing the conclusion that the fluid not likely has diffused to the chemical sensor.

The trigger sensor is a sensor for sensing a humidity of a gas in the environment of the humidity sensor, wherein the humidity sensor may preferably be arranged in the same compartment as the chemical sensor. Subject to the application, the gas to be analyzed may include a humidity different to an environmental gas. If, for example, the chemical sensor shall be used for analyzing the breath of a user exhaling at the device, then the relative humidity in the compartment increases owed to the users breath which is more humid than the environmental air. Specifically, when a signal of the humidity sensor exceeds a threshold indicating an increased humidity in the compartment, the chemical sensor reading taking may be triggered since it is assumed that the user presently not only exhales at the portable electronic device but the compartment for the chemical sensor and the humidity sensor is already filled by such breath. In another embodiment, an increase in humidity over time or an exceeding of the threshold by the humidity for a determined period in time may trigger the sampling of the chemical sensor reading given that compared to a slow environmental change in humidity a swift change in humidity may result from the user exhaling at the portable electronic device. The threshold used, as well as any other threshold used in connection with any trigger sensor, may be a fix threshold, or may be a variable threshold, or may be a threshold with a tolerance, etc.

In another embodiment [not claimed], the trigger sensor may be a temperature sensor. The chemical sensor may be comprised in a semiconductor chip in combination with an integrated circuit arrangement. The integrated circuit arrangement may be designed for preprocessing the chemical sensor readings or for evaluation of the chemical sensor readings. The temperature sensor may be arranged for measuring a temperature at least of a part of the integrated circuit arrangement. Any gas circulating over the integrated circuit may change the temperature in this area which in turn may indicate a sufficient exposure of the chemical sensor to the gas to be measured. Such temperature sensor may anyway be provided in the semiconductor chip for monitoring a temperature of active elements in the integrated circuit.

In general, the trigger sensor is integrated in the same chip together with the chemical sensor, preferably in a semiconductor chip. The semiconductor chip may be manufactured in a CMOS process and the chemical sensor structure and/or trigger sensor structures may be applied to the same semiconductor chip by a suitable manufacturing process, such as a MEMS process.

In another embodiment, the chemical sensor comprises a sensitive layer and a heater for heating the sensitive layer. In case the sensitive layer is made from metal oxide, such sensor forms a metal oxide sensor which becomes sensitive to the subject analyte when being heated to a sufficient temperature. Preferably, the heater and the sensitive layer are arranged on a thermally isolating part of the chip, such as on a bridge formed by thinning a substrate of the chip. By means of applying a temperature sensor for measuring a temperature of the sensitive layer or of the bridge, the arrangement can be used for measuring an air flow over said sensitive layer or of said bridge. Given that an air flow cools or heats the temperature of the sensitive element or the bridge, either such air flow causes a temperature deviation that can be measured by the temperature sensor, or a heating power may be taken as a measure for the air flow in case the temperature of the sensitive layer or the bridge is controlled to a constant value. The presence of a sufficient airflow may be taken as an indicator for a sufficient gas supply to the chemical sensor, for example, by the user exhaling at the access opening in the housing.

In another embodiment [not claimed], the trigger sensor may be a gyroscope or an acceleration sensor, for example. On the one hand, detecting a rotational or translational movement of the portable electronic device by the gyroscope or the accelerometer respectively may represent a sufficient shaking of the portable electronic device which may be taken as an indicator for a sufficient gas supply to the chemical sensor based on the assumption that by shaking the device the gas better diffuses into the compartment. On the other hand, the gyroscope or the accelerometer may also be used as indicator that there is not sufficient gas supplied to the chemical sensor, for example, when the access opening is orientated at a direction where the user cannot exhale at.

In a preferred embodiment, a third sensor may be provided for determining a triggering point in time for taking a chemical sensor reading. The third sensor may again be preferably one of a humidity sensor, a temperature sensor, a flow sensor, a gyroscope or a pressure sensor, wherein the third sensor preferably relies on a different measuring principle than the trigger sensor. For example, both, a humidity sensor and a temperature sensor may be used for triggering the chemical sensor reading. The signal may either individually be evaluated, for example, versus a threshold, wherein the chemical sensor reading may only be taken, when both signals exceed their associate threshold. Or the signals of the trigger sensor and the third sensor may be combined and the chemical sensor reading may be triggered when the combined signal fulfils a condition.

In another embodiment [not claimed], the trigger sensor may be represented by the chemical sensor itself, or by a cell of the chemical sensor being embodied as chemical sensor array comprising multiple sensor cells. In one example, in case an exhalation airstream of a user shall be analyzed in view of ethanol, the ethanol sensor may be embodied as one cell of the chemical sensor array. Another cell may be embodied for detecting CO2. The CO2 detecting cell may provide a signal when the user exhales at the chemical sensor. Whenever there is a sufficient concentration measured by the CO2 sensor cell, a reading of the ethanol sensor cell may be sampled under the assumption, that the chemical sensor is sufficiently exposed to the users breath. The trigger for a multi-cell chemical sensor may preferably trigger sensor readings at all sensor cells.

Preferably, a user may trigger a chemical sensor measurement by selecting a corresponding "app" or by an appropriate input at the portable electronic device.

Same or similar elements are referred to by the same reference numerals across all Figures.

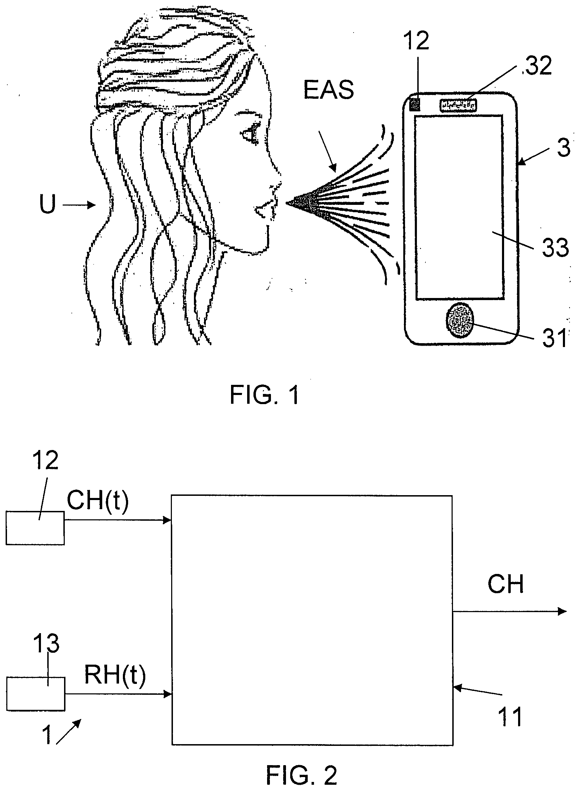

Figure 1 illustrates a usage scenario with a portable electronic device in form of a mobile phone 3 according to an embodiment of the present invention. Apart from a standard microphone 31 as an input device a chemical sensor 12 is provided next to a standard speaker 32 of the mobile phone 3. A user U blows at the mobile phone 3 such that at least a part of an exhalation air stream EAS meets the chemical sensor 12. The chemical sensor 12 in the present embodiment is represented by an Ethanol-sensor for detecting an ethanol concentration in the users breath. As a result, the detected ethanol concentration is displayed on display 33.

Figure 2 illustrates a block diagram of a portable electronic device according to an embodiment of the present invention. A chemical sensor 12 may provide an output signal CH(t) representing a conductivity of a sensitive layer being an MOx layer, however, without being heated yet. A trigger sensor 13 next to the chemical sensor 12 is embodied as a humidity sensor. The exhalation air stream of a human being is characterized by a nearly constant temperature of about 35° Celsius, and a nearly constant relative humidity of about a 100%. In general, the exhalation air stream is distinctly different in both temperature and humidity from typical environmental changes in temperature and humidity. As a result, exhaled air blown at a humidity sensor measuring the relative humidity in the compartment of the chemical sensor, will simultaneously make the relative humidity signal RH(t) rise significantly. Thus, blowing exhaled air towards such portable electronic device 1 includes a rapid change of the relative humidity signal RH(t). Hence, the humidity sensor 13 can be used as a proper means for triggering a sampling of a reading of the chemical sensor 12. Given that the chemical sensor may not permanently being heated in view of saving energy, at the point in time when a sufficient relative humidity is detected by the humidity sensor 13, a heater of the chemical sensor may be activated, and after a defined period of heating the then provided value(s) in the output signal CH(t) of the chemical sensor is taken as a sensor reading and is further used. The output signal CH(t) of the chemical sensor 12 and the relative humidity signal RH(t) are supplied by the respective sensors 12 and 13 to a control unit 11. The control unit 11 analyzes the relative humidity signal RH(t) and preferably compares the relative humidity signal to a threshold, representing, for example, a relative humidity of 85%. Whenever this threshold is exceeded at t=tₓ, the control unit 11 may accept/take the then valid output signal value CH(t=tₓ) as chemical sensor reading CH, and, for example, make this chemical sensor reading CH being displayed on a display of the mobile phone 3.

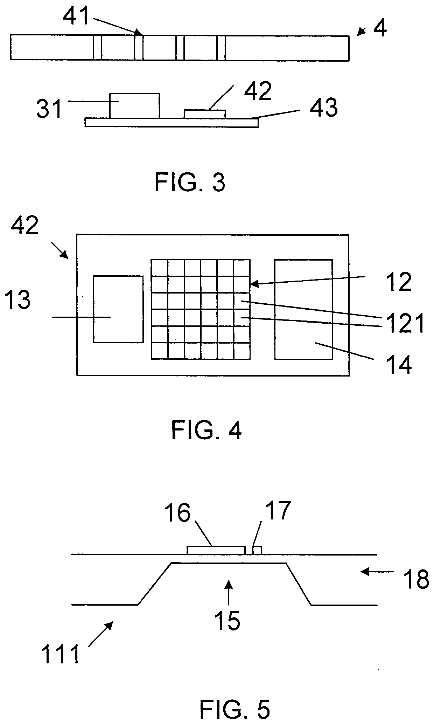

In a preferred embodiment, and as shown in the partial side cut of a lower part of a mobile phone 3 in Figure 3, a semiconductor chip 42 is arranged next to a microphone 31 on a carrier 43 of the mobile phone 3, such as a flexible circuit board or a printed circuit board. The microphone 31 and the semiconductor chip 42 are arranged next to and below an opening 41 in a housing 4 of the mobile phone 3. The opening 41 in the present example is represented by multiple bores in the housing 4. The semiconductor chip 42 carries the chemical sensor and - if available - the humidity sensor. Hence, the semiconductor chip 42 is arranged such that it is exposed to the opening 41 in order to receive an exhalation air stream directed at the opening 41.

Figure 4 illustrates a top view on a semiconductor chip 42 such as is used in the mobile phone of Figure 3. The chemical sensor 12 is represented in this semiconductor chip 42 by a chemical sensing area including multiple sensor cells 121, in the present example, thirty six sensor cells 121. In addition a humidity and/or temperature sensor 13 is integrated next to the chemical sensor array and the integrated circuitry 14.

Figure 5 illustrates a cut through a schematic individual chemical sensor 12 or one of its cells in which a recess 15 is manufactured into a substrate 18 of the semiconductor chip. On top of a resulting thin membrane, also denoted as bridge, a sensitive layer 16 is arranged, as well as a heater 17, preferably a resistive heater 17. The membrane is also denoted as micro-hotplate for the reason that such membrane to a large extent constitutes a thermally insulating structure. Hence, any heat generation by the heater 17 affects the sensitive layer 16 as desired but does not leak into the bulk. The sensitive layer 16 preferably is made from a metal oxide material such as tin oxide. The sensitive layer 16 is heated by the heater 17 prior to taking a sensor reading, and preferably during taking a sensor reading for elevating a temperature of the sensitive layer 16 to a temperature sufficient for having a catalytic reaction between the analyte/s and the sensitive layer 16 to take place at a sufficient rate and as a result, for example, for having an electrical conductivity of the sensitive layer 16 modified. Such operating temperatures may vary subject to the material used from 100° degrees Celsius to 450° degrees Celsius. The micro-hotplate, preferably fabricated in MEMS technology, enables to achieve these temperatures in the sensing area without excessive power dissipation, since only a few 10 mW of electrical power is required for heating. While such power levels may be prohibitive for continuous mode measurements in some mobile applications, single shot measurements, whereby the sensitive layer is powered up for a short time, e.g. in the order of 1 minute - and a single or a few measurement readings are taken, are possible.

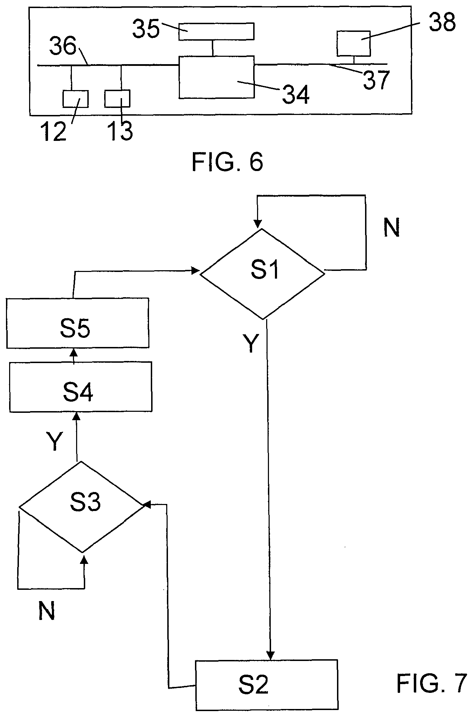

Figure 6 shows a schematic hardware oriented block diagram of a portable electronic device. Here, a software residing in a memory 38 connected to a microprocessor 34 via a system bus 37 may be executed by the microprocessor 34 on demand. Hence, the microprocessor serves as control unit for taking the chemical sensor reading at a determined point in time subject to the signal of the trigger sensor. The trigger sensor 13, presently embodied as a humidity sensor, and the chemical sensor 12 are connected to the microprocessor 34 via an input system bus 36. In addition, there is shown a wireless interface 35 of the portable electronic device.

Figure 7 illustrates a flow chart representing a method according to an embodiment of the present invention. In step S1, it is monitored if there is an active user request for conducting a chemical measurement. For example, it may be monitored, if a corresponding "app" is started by the user, or if another trigger such as the pushing of a button or a soft key is received. If there is no such request (N) the monitoring continues. If yes (Y), in step S2 a heater of the chemical sensor of the portable electronic device is activated. In step S3, it is verified if a signal of a sensor other than the chemical sensor, such as the signal of a humidity sensor, exceeds a threshold. If not (N), the signal of the trigger sensor is continued to be monitored. If yes (Y) a reading is taken at/by the chemical sensor in step S4. For example, a voltage may be supplied to a sensitive element of the chemical sensor and a conductivity of the sensitive element is determined and preferably recorded which conductivity represents a concentration of an analyte the sensitive layer is sensitive to. In case, such sensitive layer is continuously supplied by an electrical voltage, the most previous reading which may be recorded, for example, and may be taken as chemical sensor reading in step S4. The reading may then be displayed to the user in step S5 and the heater may be deactivated. In step S1 is verified again if a new request for taking a chemical measurement is received.

While there are shown and described presently preferred embodiments of the invention, it is to be distinctly understood that the invention is not limited thereto but may be otherwise variously embodied and practised.

## Claims

1. Portable electronic device, comprising
a chemical sensor (12) being sensitive to at least one analyte,
a trigger sensor (13) providing a signal (RH(t)), and
a control unit (11) configured to accept or use a measurement value supplied by the chemical sensor (12) subject to the signal (RH(t)) of the trigger sensor (13),
a semiconductor chip (42) containing the chemical sensor (12),
wherein the trigger sensor (13) includes a humidity sensor which is contained in the semiconductor chip (42).

2. Portable electronic device according to claim 1,
comprising a housing (4),
comprising a compartment in the housing (4),
which compartment comprises an access opening (41) to an environment of the portable electronic device, and
wherein the chemical sensor (12) is arranged in the compartment.

3. Portable electronic device according to claim 2,
wherein the trigger sensor (13) is arranged in the compartment.

4. Portable electronic device according to claim 1,
wherein the chemical sensor (12) comprises a sensitive layer (16) and a heater (17) for heating the sensitive layer (16),
wherein the sensitive layer (16) comprises a metal-oxide material, and
wherein the metal-oxide material includes one or more of tin oxide, zinc oxide, titanium oxide, tungsten oxide, indium oxide and gallium oxide.

5. Portable electronic device according to claim 1,
wherein the control unit (11) is adapted for triggering the sampling of the chemical sensor reading subject to the signal (RH(t)) of the trigger sensor (13) exceeding a threshold.

6. Portable electronic device according to claim 1,
comprising a third sensor supplying a signal,
wherein the third sensor is one of:
- a temperature sensor,
- a flow sensor,
- a gyroscope,
- an accelerometer,
- a microphone, and
- a pressure sensor,
wherein the control unit (11) is adapted to trigger the sampling of the chemical sensor reading subject to the signal (RH(t)) of the trigger sensor (13) and subject to the signal of the third sensor.

7. Portable electronic device according to claim 1,
wherein the chemical sensor (12) is adapted and arranged for detecting one or more of a presence and a concentration of the at least one analyte in an exhalation airflow of a user of the portable electronic device.

8. Portable electronic device according to claim 1,
which portable electronic device is one of:
a mobile phone (3),
a handheld computer,
an electronic reader,
a tablet computer,
a game controller,
a pointing device,
a photo or a video camera,
a computer peripheral.

9. Method for operating a portable electronic device containing a chemical sensor (12) being sensitive to at least one analyte and containing a trigger sensor (13), the method comprising
monitoring a signal (RH(t)) supplied by the trigger sensor (13), and
subject to the signal (RH(t)) of the trigger sensor (13) accepting or using a measurement value supplied by the chemical sensor (12), for detecting one or more of a presence and a concentration of the at least one analyte in a fluid supplied to the chemical sensor (12),
wherein the trigger sensor (13) includes a humidity sensor, and
wherein the humidity sensor and the chemical sensor (12) are contained in the same semiconductor chip (42).

10. Method according to claim 9,
wherein the signal (RH(t)) of the trigger sensor (13) is monitored in response to a user request for taking the chemical sensor reading.

11. Method according to claim 9,
wherein a heater (17) of the chemical sensor (12) is activated in response to a user request for taking the chemical sensor reading.

12. Method according to claim 9,
wherein a heater (17) of the chemical sensor (12) is activated subject to the signal (RH(t)) of the trigger sensor (13).

13. Computer program product comprising computer program code for implementing a method according to claim 9 or claim 12 when being executed on a control unit.

## Patentansprüche

1. Tragbare elektronische Vorrichtung, umfassend:
- einen chemischen Sensor (12) der zu einem Analyten empfindlich ist,
- einen Triggersensor (13) der ein Signal (RH(t)) bereitstellt, und
- eine Steuerungseinheit (11) die derart konfiguriert ist, dass sie in Abhängigkeit vom Signal (RH(t)) des Triggersensors (13) einen vom chemischen Sensor (12) gelieferten Messwert akzeptiert oder verwendet,
- einen Halbleiterchip (42), der den chemischen Sensor (12) enthält,
wobei der Triggersensors (13) einen Feuchtigkeitssensor, der im Halbleiterchip enthalten ist, einschliesst.

2. Tragbare elektronische Vorrichtung nach Anspruch 1,
umfassend ein Gehäuse (4),
umfassend eine Kammer im Gehäuse (4),
wobei die Kammer eine Zugangsöffnung (41) zu einer Umgebung der tragbaren elektronischen Vorrichtung umfasst, und
wobei der chemische Sensor (12) in der Kammer angeordnet ist.

3. Tragbare elektronische Vorrichtung nach Anspruch 2,
wobei der Triggersensor (13) in der Kammer angeordnet ist.

4. Tragbare elektronische Vorrichtung nach Anspruch 1,
wobei der chemische Sensor (12) eine empfindliche Schicht (16) und eine Heizung (17) zum Heizen der empfindlichen Schicht (16) umfasst,
wobei die empfindliche Schicht (16) ein Metalloxid-Material umfasst, und
wobei das Metalloxid-Material eines oder mehrere von: Zinnoxid, Zinkoxid, Wolframoxid, Indiumoxid und Galliumoxid einschliesst.

5. Tragbare elektronische Vorrichtung nach Anspruch 1,
wobei die Steuerungseinheit (11) zur Auslösung des Samplings der Messungen des chemischen Sensors in Abhängigkeit vom Signal (RH(t)) des Triggersensors (13), das einen Schwellenwert übersteigt, ausgestaltet ist.

6. Tragbare elektronische Vorrichtung nach Anspruch 1,
umfassend einen dritten Sensor, der ein Signal liefert, wobei der dritte Sensor einer der folgenden Sensoren ist:
- ein Temperatursensor,
- ein Strömungssensor,
- ein Gyroskop,
- ein Beschleunigungssensor,
- ein Mikrophon, und
- ein Drucksensor,
wobei die Steuerungseinheit (11) zur Auslösung des Samplings der Messungen des chemischen Sensors in Abhängigkeit vom Signal (RH(t)) des Triggersensors (13) und in Abhängigkeit vom Signal des dritten Sensors ausgestaltet ist.

7. Tragbare elektronische Vorrichtung nach Anspruch 1,
wobei der chemische Sensor (12) zur Detektierung eines oder mehrerer von: einer Anwesenheit und einer Konzentration des mindestens einen Analyten in einer Ausatem-Strömung eines Benutzers der tragbaren elektronischen Vorrichtung ausgestaltet und angeordnet ist.

8. Tragbare elektronische Vorrichtung nach Anspruch 1,
welche tragbare elektronische Vorrichtung eine der folgenden Vorrichtungen ist:
- ein Mobiltelefon (3),
- ein Taschencomputer,
- ein elektronisches Lesegerät,
- ein Tablett,
- einen Gamecontroller,
- ein Zeigegerät,
- eine Photo- oder Videokamera,
- ein Computerperipheriegerät.

9. Verfahren zum Betrieb einer tragbaren elektronischen Vorrichtung, die einen chemischen Sensor (12), der zu mindestens einem Analyten empfindlich ist, enthält, und die einen Triggersensor (13) enthält, wobei das Verfahren
die Überwachung eines vom Triggersensor (13) gelieferten Signals (RH(t)) und
in Abhängigkeit vom Signal (RH(t)) des Triggersensors (13) einen vom chemischen Sensor (12) gelieferten Messwert akzeptiert oder verwendet, um eines oder mehrere von einer Anwesenheit und einer Konzentration des mindestens einen Analyten in einem Fluid, das dem chemischen Sensor (12) zugeführt wird, zu detektieren,
umfasst,
wobei der Triggersensor (13) einen Feuchtigkeitssensor einschliesst, und
wobei der Feuchtigkeitssensor und der chemische Sensor (12) im selben Halbleiterchip enthalten sind.

10. Das Verfahren nach Anspruch 9,
wobei das Signal (RH(t)) des Triggersensors (13) als Antwort auf eine Benutzerabfrage zum Auslesen des Messwerts des chemischen Sensors überwacht wird.

11. Das Verfahren Anspruch 9,
wobei eine Heizung (17) des chemischen Sensors (12) als Antwort auf eine Benutzerabfrage zum Auslesen des Messwerts des chemischen Sensors aktiviert wird.

12. Das Verfahren nach Anspruch 9,
wobei eine Heizung (17) des chemischen Sensors (12) in Abhängigkeit vom Signal (RH(t)) des Triggersensors (13) aktiviert wird.

13. Computerprogrammprodukt umfassend Computerprogrammcode zur Implementierung eines Verfahrens nach Anspruch 9 oder Anspruch 12, wenn er auf einer Steuerungseinheit ausgeführt wird.

## Revendications

1. Dispositif électronique portable comprenant
un capteur chimique (12) sensible à au moins un analyte,
un capteur de déclenchement (13) qui fournit un signal (RH(t)), et
une unité de commande (11) configurée pour accepter ou utiliser une valeur de mesure fournie par le capteur chimique (12) dépendant du signal (RH(t)) du capteur de déclenchement (13),
une puce semi-conductrice (42) contenant le capteur chimique (12),
le capteur de déclenchement (13) incluant un capteur d'humidité qui est contenu dans la puce semi-conductrice (42).

2. Dispositif électronique portable selon la revendication 1,
comprenant une carcasse (4),
comprenant un compartiment dans la carcasse (4),
le compartiment comprenant une ouverture d'accès (41) à un environnement du dispositif électronique portable, et
le capteur chimique (12) étant arrangé dans le compartiment.

3. Dispositif électronique portable selon la revendication 2,
le capteur de déclenchement (13) étant arrangé dans le compartiment.

4. Dispositif électronique portable selon la revendication 1,
le capteur chimique (12) comprenant une couche sensible (16) et un chauffage (17) pour chauffer la couche sensible (16),
la couche sensible (16) comprenant un matériau d'oxyde de métal, et
le matériau d'oxyde de métal incluant un ou plusieurs: d'oxyde d'étain, d'oxyde de zinc, d'oxyde de titan, d'oxyde d'indium et d'oxyde de gallium.

5. Dispositif électronique portable selon la revendication 1,
l'unité de commande (11) étant adaptée à déclencher l'échantillonnage de la valeur de mesure du capteur chimique dépendant du signal (RH(t)) du capteur de déclenchement (13) qui dépasse une valeur seuil.

6. Dispositif électronique portable selon la revendication 1,
comprenant un troisième capteur fournissant un signal, le troisième capteur étant un des capteurs suivants:
- un capteur de température
- un capteur de flux,
- un gyroscope,
- un capteur d'accélération,
- un microphone, et
- un capteur de pression,
l'unité de commande (11) étant adaptée à déclencher l'échantillonnage de la valeur de mesure du capteur chimique dépendant du signal (RH(t)) du capteur de déclenchement (13) et dépendant du signal du troisième capteur.

7. Dispositif électronique portable selon la revendication 1,
le capteur chimique (12) étant adapté et arrangé pour détecter un ou plusieurs d'une présence et une concentration d'au moins un analyte dans un écoulement d'air d'exhalation d'un utilisateur du dispositif électronique portable.

8. Dispositif électronique portable selon la revendication 1,
le dispositif électronique portable étant un de:
un téléphone portable (3),
un ordinateur portable,
un lecteur électronique,
une tablette,
un contrôleur de jeu,
un dispositif de pointage,
une caméra photo ou vidéo,
une périphérie d'ordinateur.

9. Procédé pour opérer un dispositif électronique portable contenant le capteur chimique (12) qui est sensible à au moins un analyte et qui contient un capteur de déclenchement (13), le procédé comprenant
surveiller un signal (RH(t)) fournit par le capteur de déclenchement (13), et
accepter ou utiliser une valeur de mesure fournie par le capteur chimique (12) dépendant du signal (RH(t)) du capteur de déclenchement (13) pour détecter un ou plusieurs d'une présence et une concentration d'au moins un analyte dans un fluide fournit au capteur chimique (12),
le capteur de déclenchement (13) incluant un capteur d'humidité, et
le capteur d'humidité et le capteur chimique (12) étant contenus dans la même puce semi-conductrice (42).

10. Procédé selon la revendication 9,
le signal (RH(t)) du capteur de déclenchement (13) étant surveillé en réponse à une demande utilisateur pour prendre la valeur de mesure du capteur chimique.

11. Procédé selon la revendication 9,
un chauffage (17) du capteur chimique (12) étant activé en réponse à une demande utilisateur pour prendre la valeur de mesure du capteur chimique.

12. Procédé selon la revendication 9,
un chauffage (17) du capteur chimique (12) étant activé dépendant du signal (RH(t)) du capteur de déclenchement (13).

13. Produit de logiciel d'ordinateur comprenant du code de logiciel d'ordinateur pour implémenter un procédé selon la revendication 9 ou la revendication 12 quand il est exécuté dans une unité de commande.
